**Europäisches Patentamt**

**European Patent Office**

**Office européen des brevets**

(11) Publication number: **0 072 948**

**B1**

(12) **EUROPEAN PATENT SPECIFICATION**

(45) Date of publication of patent specification: **05.11.86**

(21) Application number: **82107097.6**

(22) Date of filing: **05.08.82**

(51) Int. Cl.⁴: **D 04 H 1/54,** D 06 C 23/00, A 47 L 13/16, A 61 F 13/00, A 41 B 13/02, A 61 F 5/44, A 61 L 15/00

(54) Absorbent sanitary appliance and method of producingthe same.

(30) Priority: **24.08.81 US 295719**

(43) Date of publication of application:
**02.03.83 Bulletin 83/09**

(45) Publication of the grant of the patent:
**05.11.86 Bulletin 86/45**

(84) Designated Contracting States:
**BE CH DE FR GB IT LI LU NL**

(56) References cited:
**US-A-3 737 368**
**US-A-3 965 906**
**US-A-4 059 114**
**US-A-4 100 324**
**US-A-4 154 883**
**US-A-4 200 103**

(73) Proprietor: **KIMBERLY-CLARK CORPORATION**
**401 North Lake Street**
**Neenah Wisconsin 54956 (US)**

(72) Inventor: **Whitehead, Howard A.**
**513 East Pershing Street**
**Appleton Wisconsin (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath Maximilianstrasse 58 D-8000 München 22 (DE)**

Courier Press, Leamington Spa, England.

## Description

The invention relates to an absorbent sanitary appliance having a fluid permeable wrap sheet containing fibers, the wrap sheet being provided with a continuous fusion line inset from its outer periphery, as well as to a method of producing it.

US—A—3 965 906 describes an absorbent article having an upper web of a fluid pervious non-woven material. Beneath this web of non-woven material a thermoplastic sheet is arranged in which a predetermined pattern of perforations has been fused. Between this thermoplastic sheet and a thermoplastic backing sheet an absorbent pad is arranged, which is smaller than the top and the backing sheets. Inset from the outer periphery of the absorbent article a continuous fusion line is arranged immediately adjacent to the edges of the absorbent pad. In this fusion line the thermoplastic sheet is fused to the top web and to the backing sheet, sealing the components of the absorbent appliance one to another. The fusion line provides a fluid barrier to prevent wicking and leakage from the edges of the pad. The top web of this sanitary appliance does not contain any fusible fibers so that the fused barrier line of the known appliance, for instance in case of heavy discharge, is not able to prevent the fluid from running off the surface, rather than migrating downwards into the pad. On the other hand, the fusion barrier line of the known appliance is used for sealing together the components of the appliance. Therefore, the components beneath the fusion line all loose their absorbent properties.

US—A 4 059 114 describes a disposal shield for everyday feminine hygiene, in which a fluid permeable sheet covers a web of absorbent paper fluff and fibers lined by an outer impervious sheet. The permeable shield is provided with a pattern of line embossments. These line embossments consist of discontinuous lines each one of which ends at a distance from the edges of the shield and is formed after all components of the shield have been assembled so that the lines of embossment may be fused to adjacent layers. The purpose of these lines is to prevent wicking of an absorbed fluid onto the undergarment to which the shield is adhesively attached.

US—As 4 100 324 and 4 154 883 disclose fiber webs which are provided with embossment patterns. The purpose of these patterns is to reinforce the web by fusing the fibers together in discrete locations.

In US—As 3 294 091 and 4 200 103 a thermoplastic baffle is positioned in a sanitary appliance not only on its bottom, but also along the sides so as to cover the longitudinal top edges. The baffle is then fused to the fluid permeable wrap to provide a fluid migration barrier.

Fusing has also been used to seal a napkin at its edges. Examples of such a napkin are disclosed in US—A 4 059 114. On sealing lines, however, adjacent layers must be fused together.

This will result in destruction of wicking properties of inner layers and thereby reduces absorbing volume of the appliance.

Prior art utilization of fusing fluid pervious wraps to fluid impervious baffles at the top portion of the napkin involves precise positioning of the fusing element, it must be done after assembly of a napkin. As such, it is much more difficult to properly position all of the elements to create a uniform barrier line. Also, this approach tends to damage the absorbent capacity of the absorbent layer underneath due to the application of heat necessary for fusing. Positioning of a barrier is also dependent upon precise positioning of the baffle and the location of the barrier is also predetermined by the variety of possibilities of baffle configuration and position.

The task underlying the present invention is to provide a fluid permeable sheet of the type outlined above which by simple means prevents the fluid from running off the surface of the sheet rather than migrating into or through the sheet.

This task is solved according to the invention by providing a wrap sheet which contains fusible fibers so that the fusion line forms a fluid barrier in the wrap sheet itself and that the wrap sheet is non-fusibly attached at the position of the barrier line with the other components of the appliance.

The continuous, closed fluid barrier line formed by fusing the fusible fibers in the wrap sheet, which is unfused to any other component, confines the fluid within an area spaced from the sheet edge on all sides. The fluid will, therefore, penetrate downwardly into the sheet and into the absorbent pad. Run-off of the fluid, which is particularly critical in the case of relatively viscous discharge fluids such as menses, is thus securely prevented by very simple means.

The invention also provides a simple method of producing an absorbent sanitary appliance having a fluid permeable wrap sheet, an absorbent matrix and a fluid impermeable baffle which are sealed together. This method is characterised in that a predetermined continuous, closed fluid barrier line inset from the outer periphery of the appliance is generated in the wrap sheet by fusing fusible fibers and that the wrap sheet is sealed around the other components of the sanitary appliance with the wrap sheet being non-fusibly attached at the position of the barrier line the other components.

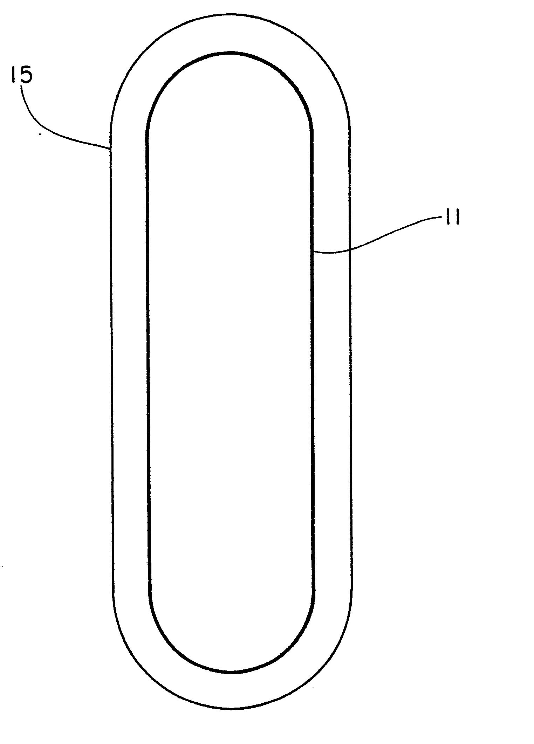

An embodiment of the invention is shown in the drawing. The only Figure shows a top view of a fluid permeable sheet according to the invention.

A sheet 15 consists of a non-woven material comprising thermoplastic fibers. A continuous fluid barrier line 11 is provided in that sheet, the line forming a closed oval which is inset from the outer periphery of the sheet 15. The line 11 is produced by embossing, e.g. by sending a web forming the sheet 15 through a pair of embossing rolls. Along line 11 the thermoplastic fibers are fused together so that it forms a barrier preventing fluid runoff from the area within the line.

Sheet 15 is used as a wrap of a sanitary appliance, such as wound dressings, diapers, incontinence garments and the like, in which the wrap contains an absorbent batt.

While sheet 15 is thermoplastic in nature, it need not be completely thermoplastic and may in fact be at least partially absorbent. One particularly useful material is disclosed in US—A 4,100,324. This patent discloses a web of material formed from a merging of air streams of thermoplastic micro-fibers and cellulosic fibers. This airlaid web has varying absorbent capabilities depending upon the amount of cellulosic material present therein.

## Claims

1. Absorbent sanitary appliance, having a fluid permeable wrap sheet containing fibers, said wrap sheet being provided with a continuous fusion line (11) inset from the outer periphery, characterised in that the wrap sheet contains fusible fibers so that the fusion line (11) forms a fluid barrier in the wrap sheet itself, and that the wrap sheet is non-fusibly attached at the position of the barrier line with the other components of the appliance.

2. Method of producing an absorbent sanitary appliance according to claim 1, having a fluid permeable wrap sheet, an absorbent matrix and a fluid impermeable baffle which are sealed together, characterised in that a predetermined continuous, closed fluid barrier line inset from the outer periphery of the appliance is generated in the wrap sheet by fusing of fusible fibers, and the wrap sheet is sealed around the other components of the sanitary appliance with the wrap sheet being non-fusibly attached at the position of the barrier line with the other components.

## Patentansprüche

1. Absorbierender Gegenstand für sanitäre Verwendung, mit einem flüssigkeitsdurchlässigen, Fasern enthaltenden Deckblatt, das einwärts des äußeren Umfangs mit einer durchgehenden Verschmelzungslinie (11) versehen ist, dadurch gekennzeichnet, daß das Deckblatt Schmelzfasern enthält, so daß die Verschmelzungslinie (11) eine Flüssigkeitssperre im Deckblatt selbst bildet und daß das Deckblatt an der Stelle der Sperrlinie nicht verschmelzend mit den anderen Bestandteilen des Gegenstandes verbunden ist.

2. Verfahren zum Herstellen eines absorbierenden Gegenstandes für sanitäre Verwendung nach Anspruch 1, der ein flüssigkeitsdurchlässiges Deckblatt, eine absorbierende Grundschicht und eine flüssigkeitsundurchlässige Sperrschicht, die miteinander verbunden sind, aufweist, dadurch Gekennzeichnet, daß eine vorherbestimmte, durchgehende, geschlossene Flüssigkeitssperrlinie in der Deckschicht einwärts des äußeren Umfanges des Gegenstandes erzeugt wird, in dem Schmelzfasern geschmolzen werden, und daß die Deckschicht derart mit den anderen Bestandteilen des Gegenstandes für sanitäre Verwendung verbunden wird, daß die Deckschicht an der Stelle der Sperrlinie nicht schmelzend mit den anderen Bestandteilen verbunden ist.

## Revendications

1. Dispositif sanitaire absorbant, comportant une feuille d'enveloppement perméable aux fluides contenant des fibres, cette feuille d'enveloppement présentant une ligne continue de réunion par fusion (11) située en retrait par rapport au pourtour extérieur, caractérisé en ce que la feuille d'enveloppement contient des fibres fusibles telles que la ligne de réunion par fusion (11) fait barrage aux fluides au sein même de la feuille d'envelloppement, et que la feuille d'enveloppement est fixée sans fusion aux autres éléments du dispositif à l'emplacement de la ligne de barrage.

2. Procédé de fabrication de dispositif sanitaire absorbant selon la revendication 1, comportant une feuille d'enveloppement perméable aux fluides, une matrice absorbante et un déflecteur imperméable aux fluides réunis hermétiquement, caractérisé en ce qu'on établit dans la feuille d'enveloppement une ligne fermée continue déterminée faisant barrage aux fluides située en retrait par rapport au pourtour extérieur du dispositif, et qu'on étanche la feuille d'enveloppement autour des autres éléments du dispositif sanitaire, la feuille d'enveloppement étant fixée sans fusion aux autres éléments à l'emplacement de la ligne de barrage.

15

11